# EUROPEAN PATENT APPLICATION

(11) **EP 3 620 785 A1**
(43) Date of publication of application: **11.03.2020**
(21) Application number: 18193226.0
(22) Date of filing: 07.09.2018
(51) Int. Cl.: G01N 27/447, C07K 1/26

(54) **ELECTROPHORESIS-BASED CHARACTERIZATION OF FC FUSION PROTEINS**

(71) Applicant: Ares Trading S.A., 1170 Aubonne (CH)
(72) Inventor: The designation of the inventor has not yet been filed
(74) Representative: Merck Serono S.A. Intellectual Property

(57) **Abstract**

The present invention provides an electrophoresis based method, in particular CGE-SDS method applied to a complex Fc fusion protein, for characterization and monitoring such Fc fusion protein in a production process.

## Description

### FIELD OF THE INVENTION

The invention described herein is directed to methods of monitoring critical attributes of Fc-fusion proteins in manufacturing such Fc-fusion proteins and in particular an assay method based on capillary gel electrophoresis.

### BACKGROUND

The use of Fc fusion proteins to improve pharmacological properties of therapeutic protein domains has increased significantly in the last decades. Method development, validation and characterization of this this class of proteins represents a unique analytical challenge due to the inherent heterogeneity of recombinant protein expression. Moreover, several physical and chemical degradation pathways can occur during manufacturing and storage that compromise protein integrity, leading to a potentially harmful, unstable product.

Previous characterization and monitoring methods for proteins often include reverse phase (RP) HPLC. However, such RP HPLC methods suffer from the drawback that for complex molecules, including Fc fusion proteins the separation on RP HPLC is not always complete. Accordingly, characterization and monitoring for impurities may be compromised.

Capillary Electrophoresis (CE)-based separation techniques, such as Capillary Gel Electrophoresis CGE -SDS, provide versatile, efficient, and fast analyses. However, CGE-SDS method characterization is generally challenging due to the inability of collecting separated fractions in order to perform species identification by direct characterization approaches.

### SUMMARY OF THE INVENTION

The present invention provides an electrophoresis based method, in particular CGE-SDS method applied to a complex Fc fusion protein, for characterization and monitoring such Fc fusion protein in a production process. The method is being developed to replace and overcome the above mentioned limitations in previous methods for monitoring species (e .g. fragments, clipped species and truncated forms), under reducing and non -reducing conditions, for complex proteins including Fc fusion proteins.

### BRIEF DESCRIPTION OF THE DRAWINGS

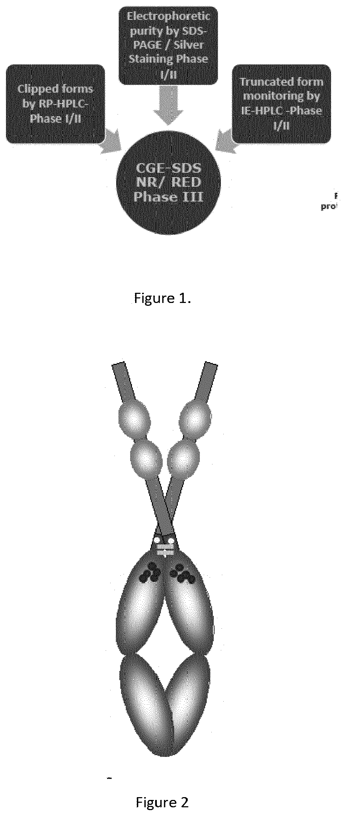

Figure 1: Schematic comparison between previous methods of monitoring and characterizing protein molecules and single CGE-SDS method.
Figure 2: Schematic representation of a Fc Fusion protei, also showing the hinge region which is prone to clipping.

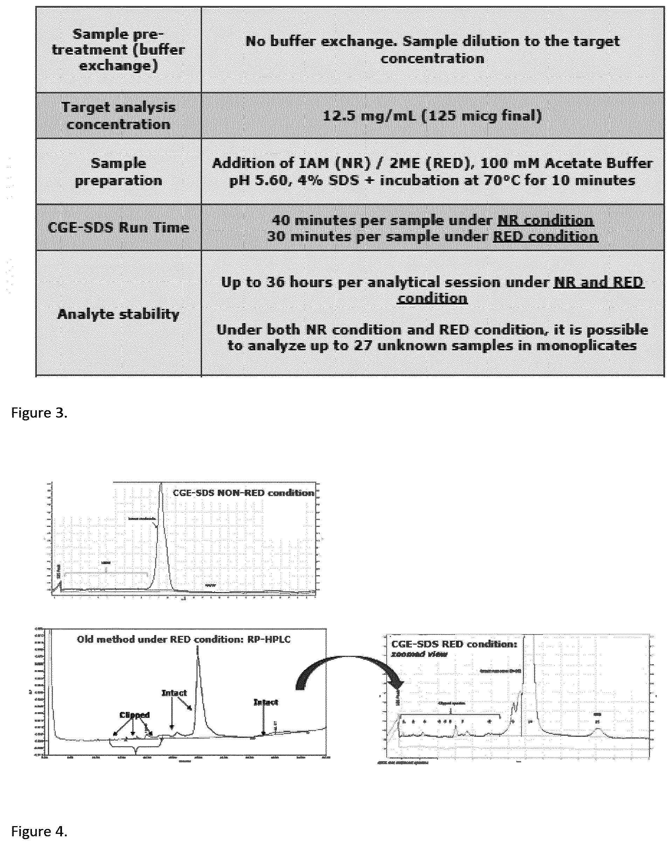

Figure 3: Schematic representation of aspects of an exemplary electrophoresis based method as described herein.
Figure 4: Shows complex profiles by CGE SDS of a Fc fusion protein, under non-reducing (NR) and reducing (RED) conditions and comparison of profile of such protein using RP-HPLC under reducing (RED) conditions where under CGE-SDS under reducing conditions the Fc fusion protein product and % clipped species could be fully indicated.
Figure 5: Schematic representation of steps in the CGE-SDS method.

### DETAILED DESCRIPTION

The present invention provides an electrophoresis based method, in particular CGE-SDS method applied to a complex Fc fusion protein, for characterization and monitoring such Fc fusion protein in a production process. The method is being developed to replace and overcome the above mentioned limitations in previous methods for monitoring species (e .g. fragments, clipped species and truncated forms), under reducing and non -reducing conditions, for complex proteins including Fc fusion proteins. The electrophoresis based, and in particular the CGE-SDS based method, provides a method that is very versatile for the monitoring and characterization in the production of complex proteins such as Fc fusion proteins.

This method is able to detect fragmentation in terms of % Low Molecular Weight (LMW) species like the pre-existing SDS PAGE silver staining method under non-reducing conditions, the % Clipped species sunder reducing conditions like the pre-existing RP HPLC method, % Purity, and free fragment of the molecule, previously monitored through iEX-HPLC.

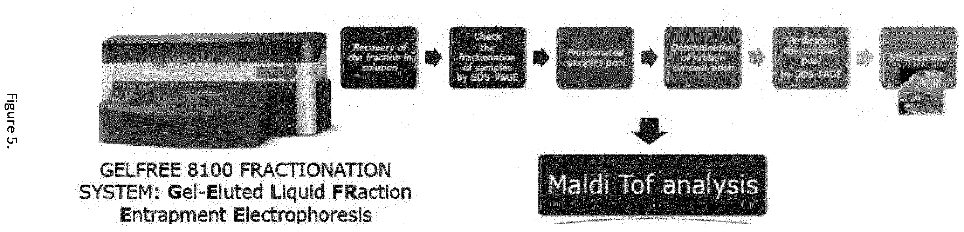

An in depth characterization was necessary to gain a thorough understanding of a complex profile. The work further extends the use of an electrophoretic fractionator system to separate various molecular species, demonstrating the high potential of combining CGE -SDS methods with SDS -PAGE and mass spectrometry, and showing the importance of establishing proper correlation of different techniques to characterize proteins. The study was composed by two main steps as described hereafter and in figure 5.

## Claims

1. An electrophoretic method for determining impurities in a complex protein sample comprising the steps of:
- diluting the sample to a protein concentration of 1 to 25 mg/ml,
- adding an alkylating agent for electrophoresis under non-reducing conditions or a reducing agent for electrophoresis under reducing conditions,
- adding electrophoretic buffer,
- incubation of sample at 50°C to 85°C for a period of 5 to 20 min,
- separate complex protein from impurities by electrophoresis of the prepared sample from previous step for a period of 20 to 60 min.
- determine presence in the sample of protein(s) or protein fragments other than the complex protein.

2. The method of claim 1, wherein the electrophoretic method is a capillary gel electrophoresis method.

3. The method of any one of claim 1 and 2, wherein the sample is diluted to 12.5 mg/ml protein.

4. The method of any one of the preceding claims, wherein the alkylating agent is iodoacetamide.

5. The method of any one of the preceding claims, wherein the reducing agent is beta mercaptoethanol.

6. The method of any one of the preceding claims, wherein the electrophoretic buffer comprises 50 to 150 mM acetate buffer and 1 to 10 % SDS at pH 5-6.

7. The method of claim 6, wherein the electrophoretic buffer comprises 100 mM acetate buffer and 4 % SDS at pH 5.6.

8. The method of any one of the preceding claims, wherein the incubation at 70°C for 10 min.

9. The method of any one of the preceding claims, wherein the electrophoresis is carried for a period of 30 to 40 min.

10. The method of any one of the preceding claims, wherein the complex protein sample is a Fc fusion protein sample

11. The method of any one of the preceding claims, wherein the method further comprises identifying and characterization of the impurities.

12. An electrophoretic method for identifying and characterizing impurities in a complex protein sample comprising the steps of:
- performing the method of any one of claims 1 to 11,
- isolating the material from each peak in the CGE profile,
- carrying out SDS-PAGE on each of the peaks isolated in the previous step, and
- identifying the protein or protein fragment obtained in the previous step.

13. The method of claim 12, wherein the method comprises capillary gel electrophoresis followed by SDS-PAGE electrophoresis.

14. The method of any one of claims 12 or 13, wherein the complex protein sample is a Fc fusion protein sample.
